# EUROPEAN PATENT SPECIFICATION

(11) **EP 1 275 353 B1**
(45) Date of publication and mention of the grant of the patent: **07.02.2007**
(21) Application number: 02023220.3
(22) Date of filing: 26.05.2000
(51) Int. Cl.: A61F 2/06, A61B 17/11

(54) **Anastomosis member for anastomosis of blood vessels**
Vorrichtung zur Gefässeanastomose
Elément d'anastomose de vaisseaux sanguins

(30) Priority: 26.05.1999 JP 14727899; 16.07.1999 JP 20331899
(43) Date of publication of application: 15.01.2003
(62) Divisional of application: 00111401.6
(73) Proprietor: Nec Tokin Corporation, Sendai-shi, Miyagi (JP)
(72) Inventor: Sato, Akira, Sendai-shi, Miyagi (JP); Suzuki, Masao, c/o NEC TOKIN Corporation, Sendai-shi, Miyagi (JP); Furukawa, Akihisa, c/o NEC TOKIN Corporation, Sendai-shi, Miyagi (JP)
(74) Representative: Hofer, Dorothea

(56) References cited:
- WO-A-00/27313
- WO-A-99/55254
- US-A- 5 254 127
- US-A- 5 540 701
- US-A- 5 824 053
- US-A- 6 017 362

## Description

### Background of the Invention:

This invention relates to an anastomosis member for anastomosing blood vessels.

An anastomosis member serves to perform the anastomosis of two normally distinct hollow organs, such as blood vessels, to form a passage therethrough.

In a conventional surgical operation, the anastomosis of the blood vessels is generally carried out by suturing with a needle and a suture filament. When the blood vessels are anastomosed or joined to each other, a blood flow must temporarily be interrupted. If an increased number of sites are required to be anastomosed, the time of interrupting the blood flow is unfavorably extended.

In the surgical operation of a living body, it is required to use auxiliary means such as extracorporeal circulation or controlled hypothermia if it is presumed that the time of interrupting the blood flow exceeds an allowable time for the living body. It is difficult to suture the blood vessels under arteriosclerosis with the needle and the suture filament if those vessels have calcification. In case where the blood vessels are fragile, they must be reinforced to avoid the risk.

The technique of using a stent in the anastomosis of the blood vessels with heavy calcification is reported. In this technique, an artificial blood vessel is inserted into a host blood vessel to partially overlap each other. The stent is retained in the blood vessels at the overlapping portion to press-fit the blood vessels to each other. Thus, the anastomosis is carried out. For example, a type of the stent is disclosed in U.S. Patent No. 6,017,362.

In the technique of anastomosing the blood vessels by the use of the stent, the blood vessels are simply press-fitted by the elasticity of the stent and may be undesirably released from each other by, for example, the beat of the artery. Thus, the anastomosis by the use of the existing stent is insufficient in fixing or engaging force and in reliability.

Document US 5,540,701 A relates to an anastomosis member comprising two generally cylindrical bodies being connected through a connecting member, wherein each generally cylindrical body both has a non-deployed state and a deployed state to become expanded radially.

The US 5,254,127 A describes a flexible body, being either in the form of a compressible hollow cylinder or in the form of a flat sheet which can be rolled to a cylindrical body, wherein the flexible body has a plurality of micro miniature barbs on a surface thereof.

Furthermore, the US 5,824,053 A describes a stent comprising a helical mesh coil with a particular lattice structure, wherein the mesh coil is formed into a generally cylindrical body through a plurality of turns.

### Summary of the Invention:

It is therefore a technical object of this invention to provide an anastomosis member capable of safely and quickly carrying out the anastomosis of blood vessels and to provide an anastomosis method using the anastomosis member.

It is another object of this invention to provide an anastomosis member improved in reliability by the use of a technique of applying an engaging force only to an adventitia of a blood vessel which is stronger than an intima of the blood vessel and to provide an anastomosis method using the anastomosis member.

It is still another object of this invention to provide an anastomosis member capable of carrying out the anastomosis of blood vessels substantially equal in outer diameter and in inner diameter and to describe for purposes of illustration, an anastomosis method using the anastomosis member.

According to this invention, there is provided an anastomosis member as defined in claim 1. Preferred embodiments are set farth in the subclaims.

Preferably, the anastomosis member has a plurality of the generally cylindrical bodies and at least one connecting member connecting the generally cylindrical bodies to one another.

Preferably, the anastomosis member has an elasticity so as to be compressed and expanded in diameter.

The anastomosis member has a stress-strain characteristic including a plurality of different kinds of regions at least corresponding to a low-rigidity part deformable along the curvatures of the first and the second blood vessels to be tightly fitted thereto, and a spring region which is compressible and self-expandable in diameter.

Preferably, the generally cylindrical body comprises a plurality of the plate members connected to one another in a zigzag pattern.

Preferably, the generally cylindrical body comprises at least one plate member wound into a helical shape

Preferably, the generally cylindrical body comprises a plurality of the plate members connected to one another in a lattice pattern.

Preferably, the generally cylindrical body comprises a plurality of the plate members connected to one another in a rhombic pattern.

Preferably, the generally cylindrical body comprises a plurality of the plate members arranged in parallel to one another and a plurality of connecting members connecting the plate members to one another.

Preferably, each of the connecting members is an elastic wire member.

Preferably, the plate member is made of a stainless steel plate or a shape memory alloy selected from a TiNi alloy and a beta Ti alloy.

### Brief Description of the Drawing:

Fig. 1 is a perspective view of an astomosis member, useful for the understanding of this invention;
Fig. 2 is a perspective view of an astomosis member, useful for the understanding of this invention;
Fig. 3 is a perspective view of an anastomosis member, useful for the understanding of this invention;
Fig. 4 is a perspective view of an anastomosis member according to an embodiment of this invention;
Fig. 5 is a perspective view of an anastomosis member, useful for the understanding of this invention;
Fig. 6 is a perspective view of an anastomosis member, useful for the understanding of this invention;
Fig. 7 is a perspective view of an anastomosis member, useful for the understanding of this invention;
Fig. 8 is a perspective view of an anastomosis member according to another embodiment of this invention;
Fig. 9 is a development view showing the anastomosis member of Fig. 8;
Fig. 10 is a transversal sectional view illustrating a first anastomosis example of blood vessels using the anastomosis member of Fig. 1;
Fig. 11 is a transversal sectional view illustrating a second anastomosis example of blood vessels using the anastomosis member of Fig. 5;
Fig. 12 is a transversal sectional view illustrating a third anastomosis example using a two-piece anastomosis device;
Fig. 13 is a transversal sectional view illustrating a fourth anastomosis example using a three-piece anastomosis device;
Fig. 14 is a deployed plan view of an anastomosis member, useful for the understanding of this invention;
Fig. 15 is a transversal sectional view illustrating a fifth anastomosis example using a composite anastomosis device; and
Fig. 16 is a cross-sectional view taken along in a line XVI-XVI in Fig. 15.

### Description of the Preferred Embodiments:

Now, description will be made about several reference examples and preferred embodiments of this invention with reference to the drawing.

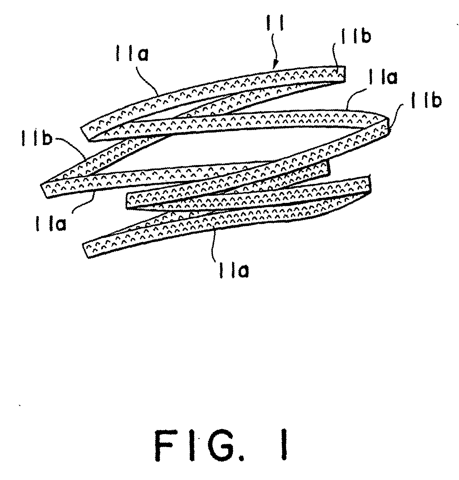

At first referring to Fig. 1, an anastomosis member 11 has a generally cylindrical body comprising a plurality of plate members or sections 11a connected to one another in a zigzag pattern extending along a cylindrical surface of an imaginary tube. Each of the plate members 11a has opposite surfaces having a plurality of protrusions 11b formed thereon.

The anastomosis member 11 is prepared in the following manner. At first, the plate members 11a are formed. For example, each of the plate members 11a comprises a strip-like metal plate having a thickness of 0.2mm and a width of 2.0mm. Each of the protrusions 11b has a height of 70µm and a diameter of 30 µm. The protrusions 11b are arranged at a pitch of 0.3mm on the opposite surfaces of each plate member 11a. Thereafter, the plate members 11a are connected at their ends to one another by welding to extend in a zigzag pattern and to form the generally cylindrical body as the anastomosis member 11.

Alternatively, the anastomosis member 11 may be prepared from a sheet-like plate material. At first, the protrusions 11b are formed on the plate material. By the use of a laser or wire electric discharge, the plate material is cut into a zigzag pattern and then rolled into a cylindrical shape. Finally, rolled ends are welded to each other.

For example, the anastomosis member 11 thus prepared forms an imaginary tube having a cylindrical diameter of 8mm and an axial length of 10mm. The anastomosis member 11 is elastically variable in shape and in diameter.

The anastomosis member 11 can be produced in a different manner. Specifically, the plate member 11a is made of a stainless steel material (SUS316) subjected to annealing and having a low rigidity. For example, the anastomosis member 11 has a cylindrical diameter of 6.5mm and an axial length of 10mm.

Alternatively, the anastomosis member 11 may be produced by bending a single long strip-like stainless steel plate in a zigzag pattern having a plurality of short strip-like plate sections 11a and then rounding the zigzag pattern of the stainless steel plate into a cylindrical shape.

The anastomosis member 11 has a zigzag pattern. However, the anastomosis member 11 may have various other patterns which will be described below.

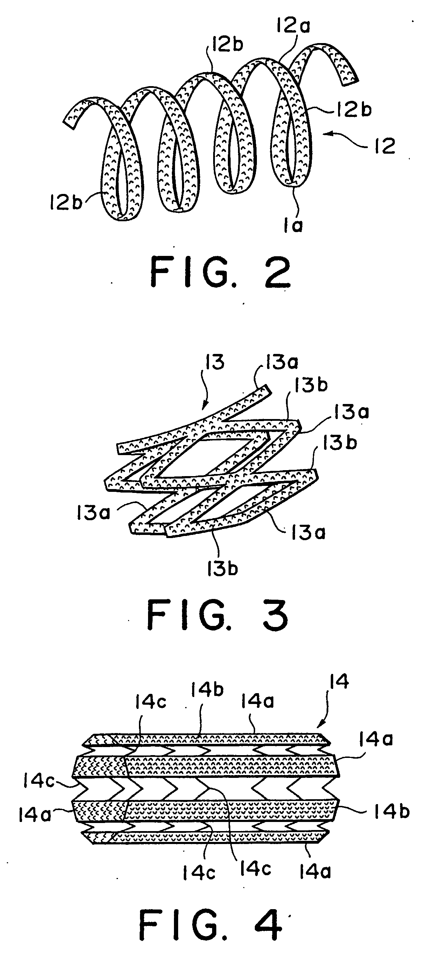

Referring to Fig. 2, an anastomosis member 12 has a generally cylindrical body comprising a helical coil formed by bending or winding a single long strip-like plate member 12a. Like in the first embodiment, the plate member 12a is provided with a plurality of protrusions 12b formed on opposite surfaces thereof. Instead of the single plate member 12a as the helical coil having a predetermined length, the anastomosis member 12 may comprise a plurality of helical coil elements which are connected in series to one another.

Referring to Fig. 3, an anastomosis member 13 has a generally cylindrical body comprising a plurality of strip-like plate members 13a connected to one another in a rhombic or a lattice pattern. Like in the foregoing embodiments, the plate member 13a is provided with a plurality of protrusions 13b formed on opposite surfaces thereof.

Referring to Fig. 4, an anastomosis member 14 according to an embodiment of this invention has a generally cylindrical body comprising a plurality of strip-like plate members 14a equal in length and arranged in parallel to one another with an angular space kept from one another. These plate members 14a are connected by a plurality of wire connecting members 14c to form the generally cylindrical body as the anastomosis member 14. Like in the foregoing reference examples, the plate member 14a is provided with a plurality of protrusions 14b formed on its opposite surfaces.

For example, each of the plate members 14a comprises a stainless steel plate subjected to annealing and having a low rigidity. For example, each of the plate members 14a has an axial length of 10mm, a thickness of 0.2mm, and a width of 1.2mm. The anastomosis member 14 has a diameter of 8mm. Each of the connecting members 14c comprises a stainless steel wire (SUS304WP) having a length of 2mm and a diameter of 0.2mm. In this embodiment, the connecting members 14c comprise spring wires which are equal in length to each other and each of which is bent to form an angled portion having an acute angle.

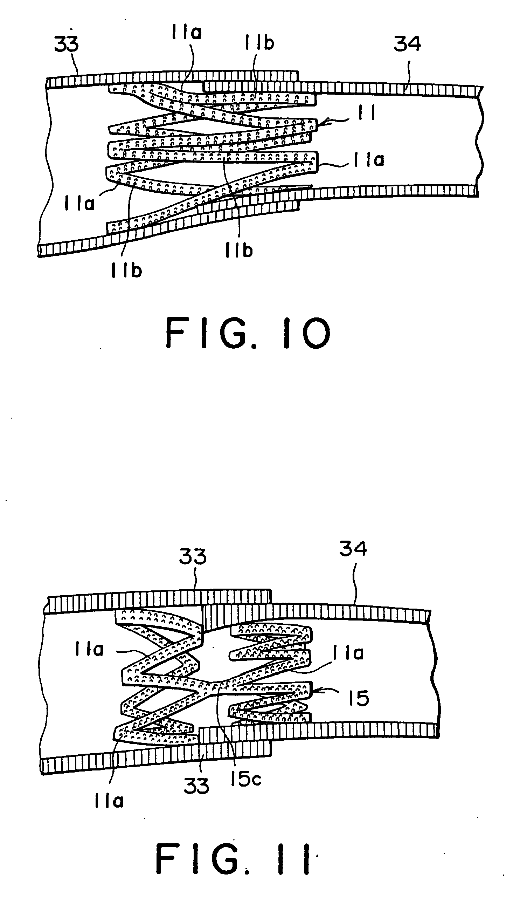

Specifically, the anastomosis member 14 has a low-rigidity part (plate members 14a) deformable along the curvatures of first and second blood vessels (Fig. 10, 33 and 34) to be tightly fitted thereto, and a self-expandable spring part (connecting members 14c). Thus, the anastomosis member 14 has a stress-strain characteristic including at least two different kinds of regions.

The protrusions 14b are formed on the opposite surfaces of each plate member 14a. The plate members 14a and the connecting members 14c are welded to each other to form the generally cylindrical body as the anastomosis member 14. The anastomosis member 14 can be expanded and compressed by changing the angles of the angled portions of the connecting members 14c.

As described above, the plate members 14a and the connecting members 14c are different from each other in stress-strain characteristic. The plate members 14a are soft and low in rigidity. Therefore, the anastomosis member 14 is readily deformable in conformity with the curvatures of the first and the second blood vessels without local pressure concentration in the first and the second blood vessels. Thus, the anastomosis member 14 can uniformly apply the pressure upon the first and the second blood vessels. Because of presence of the protrusions 14b, the anastomosis member 14 can be engaged with the first and the second blood vessels with a large frictional force.

It is noted here that the shape of the anastomosis member 14 is not restricted to that illustrated in Fig. 4. The connecting member 14c can be formed into a rhombic shape or any other appropriate shape as far as the connecting members 14c can hold the plate member 14a and are self-expandable. The plate member 14a is not restricted to the shape described in this embodiment but may have any other appropriate shape matching the configurations of the first and the second blood vessels.

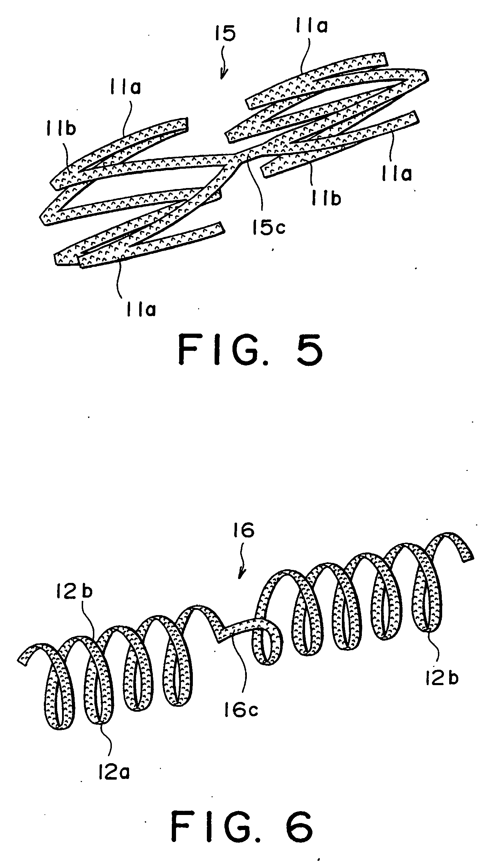

Referring to Fig. 5, an anastomosis member 15 comprises a pair of generally cylindrical bodies connected by a connecting portion 15c. Each of the generally cylindrical bodies is similar in structure to the generally cylindrical body of the anastomosis member 11 in Fig. 1. The connecting portion 15c is made of a material same as that of plate members 11a.

Referring to Fig. 6, an anastomosis member 16 comprises a pair of generally cylindrical bodies connected by a connecting portion 16c. Each of the generally cylindrical bodies is similar in structure to the generally cylindrical body of the anastomosis member 12 in Fig. 2. The connecting portion 16c is made of a material same as that of plate members 12a.

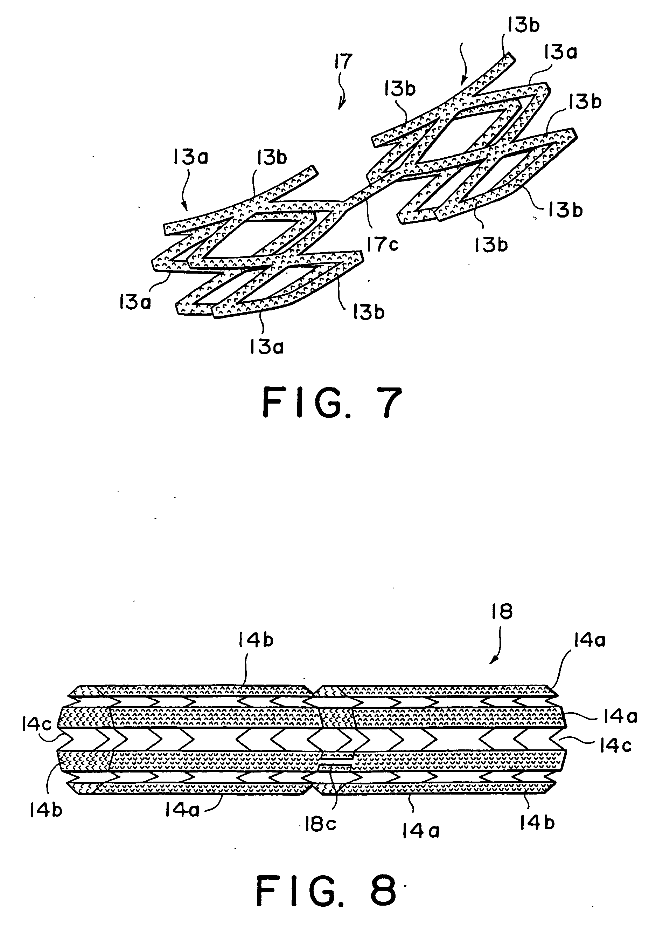

Referring to Fig. 7, an anastomosis member 17 comprises a pair of generally cylindrical bodies connected by a connecting portion 17c. Each of the generally cylindrical bodies is similar in structure to the generally cylindrical body of the anastomosis member 13 in Fig. 3. The connecting portion 17c is made of a material same as that of plate members 13a.

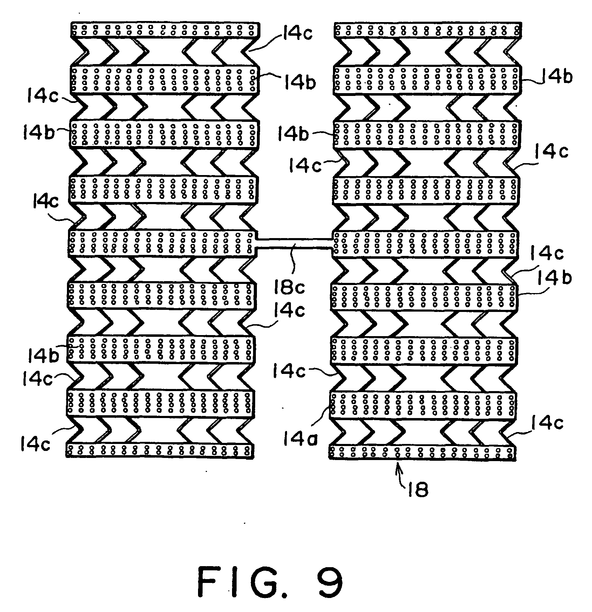

Referring to Figs. 8 and 9, an anastomosis member 18 according to another embodiment of this invention comprises a pair of generally cylindrical bodies connected by a connecting portion 18c. Each of the generally cylindrical bodies is similar in structure to the generally cylindrical body of the anastomosis member 14 in Fig. 4. The connecting portion 18c is made of a material same as that of plate members 14a. The connecting portion 18c is smaller in width than each plate member 14a.

In the foregoing embodiments, the plate members 11a, 12a, 13a, and 14a are provided with the protrusions 11b, 12b, 13b, and 14b formed on both of the opposite surfaces thereof, respectively. Alternatively, the protrusions may be formed on only one of the opposite surfaces thereof. Each of the anastomosis members 11 through 18 is not restricted to the pattern described in each of the foregoing embodiments but may have any appropriate pattern as far as the diameter can flexibly be changed.

As a material of each of the plate members 11a, 12a, 13a, and 14a in the foregoing embodiments, use may be made of a stainless steel plate, a TiNi alloy and a TiNi-X alloy (X = Cr, V, Cu, Fe, Co, etc) having superelasticity at a living body temperature. Furthermore, use may also be made of a wide variety of shape memory alloys, such as a Cu-based alloy and a Fe-based alloy, as well as a beta Ti alloy. Taking the biocompatibility and the toxicity into consideration, the opposite surfaces of the plate members 11a, 12a, 13a, and 14a may be coated with titanium or the like.

Now, description will be made of several specific examples of the anastomosis of the first and the second blood vessels.

Referring to Fig. 10, a first example of the anastomosis will be described. Herein, the anastomosis is carried out by the use of the anastomosis member 11 illustrated in Fig. 1. In the following description, the similar parts are designated by like reference numerals. It will be noted here that the size of the anastomosis member 11 in this example is slightly different from that mentioned in conjunction with Fig. 1.

As illustrated in Fig. 10, the anastomosis member 11 is arranged over a host blood vessel 33 as the first blood vessel and an artificial blood vessel 34 as the second blood vessel. An end portion of the artificial blood vessel 34 is inserted into an end portion of the host blood vessel 33. The host and the artificial blood vessels 33 and 34 overlap each other to form an anastomosed site. The anastomosis member 11 is arranged in contact with intimae of the host and the artificial blood vessels 33 and 34.

Since the anastomosis member 11 comprises the plate members 11a connected in a zigzag pattern, the anastomosis member 11 can be brought into contact with the host and the artificial blood vessels 33 and 34 to expand the host and the artificial blood vessels 33 and 34 independently of each other. Because the protrusions 11b provide a large frictional force, the anastomosis member 11 can be securely engaged with the intimae of the host and the artificial blood vessels 33 and 34. Therefore, even under the beat of the artery, the dislocation of the host and the artificial blood vessels 33 and 34 with respect to each other can be avoided by the use of the anastomosis member 11.

By a hand of a surgeon, the anastomosis member 11 is inserted into lumens of the host and the artificial blood vessels 33 and 34. For example, if the artificial blood vessel 34 has a diameter of 6mm, the anastomosis member 11 is compressed to a reduced diameter of 6mm or less and then inserted into the lumen of the artificial blood vessel 34. Furthermore, the anastomosis member 11 is inserted also into the lumen of the host blood vessel 33. The host and the artificial blood vessels 33 and 34 are made to approach each other to form an overlapping portion. At this time, the anastomosis member 11 is self-expanded from a compressed state. Therefore, the anastomosis can be quickly and easily carried out.

From the foregoing description, it will readily be understood that the anastomosis members 12, 13, and 14 illustrated in Figs. 2 through 4 may also be used instead of the anastomosis member 11. In this case also, the end portions of the host and the artificial blood vessels 33 and 34 can be expanded independently of each other so that the anastomosis is reliably carried out.

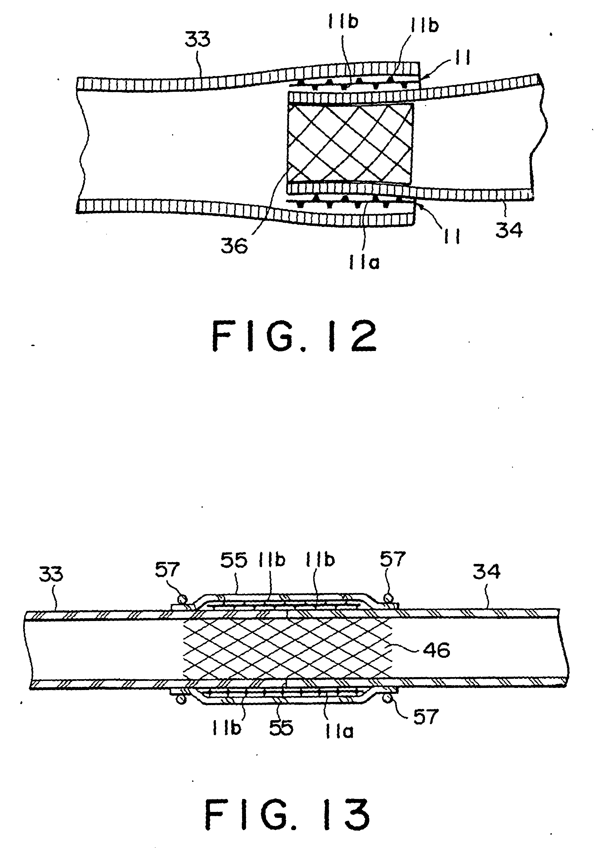

Referring to Fig. 11, a second example of the anastomosis will be described. Herein, the anastomosis is carried out by the use of the anastomosis member 15 illustrated in Fig. 5. In the following description, similar parts are designated by like reference numerals. It will be noted here that the size of the anastomosis member 15 in this example is slightly different from that mentioned in conjunction with Fig. 5.

As illustrated in Fig. 11, the anastomosis member 15 is attached to the host blood vessel 33 and the artificial blood vessel 34. The anastomosis member 15 is arranged over the lumens of the host and the artificial blood vessels 33 and 34 to be anastomosed. The end portion of the artificial blood vessel 34 is inserted into the end portion of the host blood vessel 33.

When the anastomosis member 15 is arranged in the host and the artificial blood vessels 33 and 34, the anastomosis member 15 can expand the end portions of the host and the artificial blood vessels 33 and 34 independently of each other. Thus, the anastomosis is reliably carried out.

From the foregoing description, it will readily be understood that the anastomosis members 16, 17, and 18 illustrated in Figs. 6 through 8 may also be used instead of the anastomosis member 15. In this case also, the end portions of the host and the artificial blood vessels 33 and 34 can be engaged by the anastomosis member 15 to be expandable independently of each other. Thus, the anastomosis is reliably carried out.

Referring to Fig. 12, a third example of the anastomosis will be described. Herein, the anastomosis is carried out by the use of a two-piece anastomosis device comprising a combination of the anastomosis member 11 in Fig. 1 and a woven tubular stent 36. In the following description, similar parts are designated by like reference numerals. It will be noted here that the size of the anastomosis member 11 in this example is slightly different from that mentioned in conjunction with Fig. 1.

As illustrated in Fig. 12, the anastomosis member 11 is arranged in an anastomosed site between the host blood vessel 33 and the artificial blood vessel 34. The end portion of the artificial blood vessel 34 is inserted into the end portion of the host blood vessel 33. At the anastomosed site, the end portions of the host and the artificial blood vessels 33 and 34 overlap each other with the anastomosis member 11 interposed therebetween. In the lumen of the artificial blood vessel 34 at the anastomosed site, the stent 36 is arranged.

For example, the stent 36 comprises a shape memory alloy such as a TiNi alloy having a cylindrical shape and a lattice pattern. For example, the stent 36 is designed to have a cylindrical shape with a final expanding diameter of 7mm. The stent 36 has an axial length of 10mm. The stent 36 has superelasticity at and around the body temperature of the living body.

As illustrated in Fig. 12, the stent 36 expands the artificial blood vessel 34 outwards in a radial direction. The stent 36 is inserted into the lumen of the artificial blood vessel 34 having an outer diameter of 6mm and an inner diameter of 5mm. Then, the artificial blood vessel 34 is inserted into the host blood vessel 33 with the anastomosis member 11 interposed between the host and the artificial blood vessels 33 and 34.

The host and the artificial blood vessels 33 and 34 and the anastomosis member 11 are press-fitted by the stent 36. At this time, the protrusions 11b are engaged with the intima of the host blood vessel 33 and the adventitia of the artificial blood vessel 34. Thus, the host and the artificial blood vessels 33 and 34 are prevented from being dislocated even under the beat of the artery. Thus, the anastomosis is reliably carried out.

The stainless steel plate of the anastomosis member 11 is not restricted to SUS316 but may be any other appropriate product having a low rigidity and a flexibility. The material and the shape of the stent 36 are not restricted to those given in this embodiment but may be appropriately selected taking into account the sizes of the host blood vessel 33 and the artificial blood vessel 34 as well as an expanding force of the stent 36.

Referring to Fig. 13, a fourth example of the anastomosis will be described. Herein, the anastomosis is carried out by the use of a three-piece anastomosis device comprising a combination of the anastomosis member 11 in Fig. 1, a woven tubular stent 46, and an additional blood vessel 55. In this example also, the anastomosis member 11 serves as a securing member for securing the two blood vessels in the anastomosed state. In the following description, similar parts are designated by like reference numerals. It will be noted here that the size of the anastomosis member 11 in this example is slightly different from that mentioned in conjunction with Fig. 1.

The anastomosis member 11 is attached to the host and the artificial blood vessels 33 and 34. An end face of the artificial blood vessel 34 is abutted to an end face of the host blood vessel 33. The anastomosis member 11 is arranged on outer surfaces of the host and the artificial blood vessels 33 and 34 to extend over both of the host and the artificial blood vessels 33 and 34. The stent 46 is arranged in the lumens of the host and the artificial blood vessels 33 and 34 at an area corresponding to the anastomosis member 11.

Furthermore, the additional blood vessel 55 is arranged around the host and the artificial blood vessels 33 and 34 to cover the anastomosis member 11. The anastomosis member 11 and the additional blood vessel 55 are engaged with each other by the protrusions 11b. The opposite ends of the additional blood vessel 55 are fastened to the host and the artificial blood vessels 33 and 34 by a filament 57 or a strap as a fastening member. The protrusions 11b may be formed only on one surface of the anastomosis member 11 to be engaged with the host and the artificial blood vessels 33 and 34.

More specifically, the anastomosis member 11 comprises a strip-like plate member 11a made of a stainless steel plate (SUS316) subjected to annealing and having a low rigidity. The plate member 11a has a thickness of 0.25mm. Each protrusion 11b has a height between 65 and 75µm and a diameter of 30µm. The protrusions 11b are formed at a pitch of 0.2mm.

In this example of the anastomosis, the anastomosis member 11 has an inner diameter of 8mm, an axial length of 10mm, and a width of 0.4mm. The anastomosis member 11 is flexibly variable in shape and in cylindrical diameter.

The stent 46 is made of a material similar to that of the stent 36 illustrated in Fig. 12 and has a lattice pattern. For example, the stent 46 is designed to have a cylindrical shape with a final expanding diameter of 7mm and an axial length of 20mm.

In the anastomosis, one axial half of the stent 46 is inserted into the lumen of the artificial blood vessel 34, for example, having an outer diameter of 8mm and an inner diameter of 6mm. Then, the host and the artificial blood vessels 33 and 34 are abutted to each other. Simultaneously, the other axial half of the stent 46 is inserted into the lumen of the host blood vessel 33. The anastomosis member 11 preliminarily arranged on the outside of the artificial blood vessel 34 is positioned to extend over the outer surfaces of both of the host and the artificial blood vessels 33 and 34. Likewise, the additional blood vessel 55 arranged on the outside of the artificial blood vessel 34 is positioned around the anastomosis member 11. The additional blood vessel 55 has an inner diameter of 8.1 mm.

The opposite ends of the additional blood vessel 55 are fastened by the filament 57 as the fastening member to the outer surfaces of the host and the artificial blood vessels 33 and 34. The host and the artificial blood vessels 33 and 34 and the anastomosis member 11 are press-fitted by an internal pressure applied by the stent 46 and an external pressure applied by the additional blood vessel 55 fastened by the filament 57. At this time, the protrusions 11b are engaged with the host and the artificial blood vessels 33 and 34. Thus, the host and the artificial blood vessels 33 and 34 are prevented by the anastomosis member 11 from being dislocated with respect to each other even under the beat of the artery. Therefore, the anastomosis is reliably carried out. Since the additional blood vessel 55 and the filament 57 are fastened to each other, blood leakage from the anastomosed site to the outside is prevented.

The anastomosis in the above-mentioned example can be manually and quickly carried out by a surgeon so that the time of interrupting the blood flow can be shortened.

In this example of the anastomosis, the engaging force of the protrusions 11b acts only on the adventitia of each blood vessel which is relatively strong as compared with the intima. Thus, the load upon the host and the artificial blood vessels 33 and 34 is small as compared with the case where the inner surfaces are used in engagement. Furthermore, the host and the artificial blood vessels 33 and 34 need not overlap each other so that the anastomosis is possible even if the host and the artificial blood vessels 33 and 34 are substantially equal in diameter to each other. The anastomosis member 11 can relatively flexibly cope with the difference in size between the host and the artificial blood vessels 33 and 34.

The anastomosis member 11 is not restricted to the pattern described above but may have any pattern as far as the cylindrical diameter is flexibly variable. The material of the anastomosis member 11 is not restricted to the stainless steel plate but may be any appropriate material having a low rigidity and a flexibility.

The stent 46 is not restricted to the shape described above but may have any shape as far as an appropriate expanding force is provided so as to engage the host and the artificial blood vessels 33 and 34 and the anastomosis member 11 with one another. For example, use may be made of various kinds of stents typically used for expanding constricted portions of blood vessels.

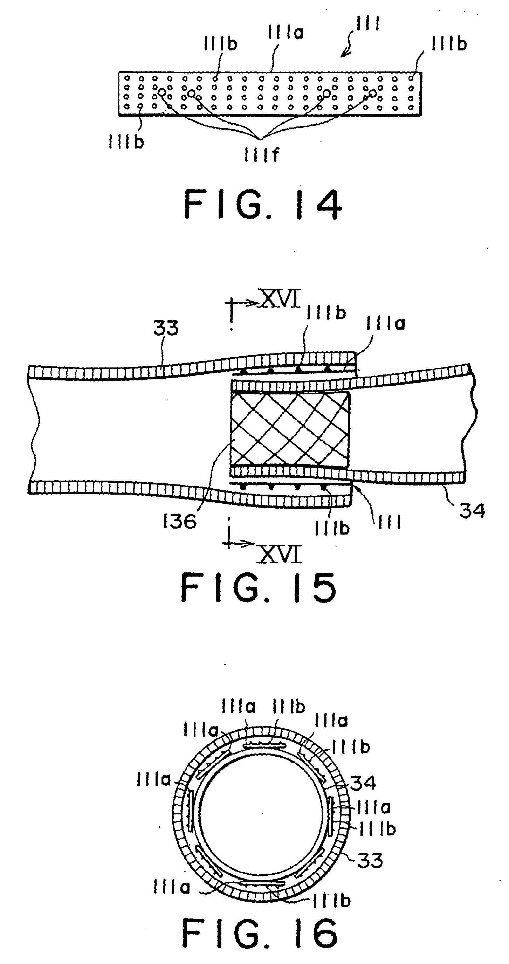

Referring to Fig. 14, an anastomosis member 111 comprises a plurality of plate members 111a although only one is illustrated in the figure. Each plate member 111a is provided with a plurality of protrusions 111b formed on only one of its opposite surfaces. The plate member 111a is substantially similar to the plate member 11a illustrated in Fig. 1 except that a plurality of through holes 111f are formed along its center line parallel to a longitudinal direction thereof. The through holes 111f allow a suture filament (not shown) to pass therethrough. The plate members 111a are sutured to the artificial blood vessel 34 by the suture filament passing through the through holes 111f.

Referring to Figs. 15 and 16, a fifth example of the anastomosis will be described. Herein, the anastomosis is carried out by the use of a composite anastomosis device comprising a combination of the anastomosis member 111 in Fig. 14 and a woven tubular stent 136. The stent 136 is substantially similar to the stent 36 illustrated in Fig. 12.

The plate members 111a are sutured to the artificial blood vessel 34 with their surfaces having the protrusions 111b faced outward. In the example being illustrated in Fig. 16, eight plate members 111a are arranged on the outer surface of the artificial blood vessel 34 to form an octagonal shape around the center axis of the artificial blood vessel 34.

As illustrated in Fig. 15, the stent 136 is inserted into the lumen of the artificial blood vessel 34. The end portion of the artificial blood vessel 34 is inserted into the end portion of the host blood vessel 33. At this time, the plate members 111a are arranged between the blood vessels 33 and 34 overlapping each other. The protrusions 111b formed on the one surface of the plate members 111a sutured to the artificial blood vessel 34 are faced to the inner surface of the host blood vessel 33. The host blood vessel 33 and the plate members 111a are press-fitted by a pressing force of the stent 136 and the host blood vessel 33 is engaged by the protrusions 111b. Thus, the host and the artificial blood vessels 33 and 34 are prevented from being dislocated with respect to each other. Thus, the anastomosis is reliably carried out.

The shape and the number of the plate members 111a are not restricted to those given in this embodiment but may be appropriately selected so that the host and the artificial blood vessels 33 and 34 are reliably engaged by the protrusions 111b.

In the foregoing, description is directed to the anastomosis of the host blood vessel 33 and the artificial blood vessel 34 inserted into the host blood vessel 33. However, this invention is also applicable to the case where the host blood vessel 33 is inserted into the artificial blood vessel 34 in dependence upon the condition of the host blood vessel 33. It will readily be understood that this invention is also applicable to the case where the first and the second blood vessels are both living blood vessels.

As described above, according to this invention, the anastomosis member having protrusions are arranged at the anastomosed site of the first and the second blood vessels so that the engaging force for engaging the first and the second blood vessels can be increased. The anastomosis member can achieve the safe anastomosis not only in a normal blood vessel but also in a diseased blood vessel. Furthermore, the anastomosis of the first and the second blood vessels can be quickly carried out.

The anastomosis member of this invention is highly reliable because the protrusions can be formed so that the engaging force acts only on the adventitia of each blood vessel which is stronger than the intima.

The anastomosis member can be attached to the first and the second blood vessels which are abutted at their end faces to each other with the stent inserted into the lumens of the first and the second blood vessels. Therefore, it is possible to carry out the anastomosis for the first and the second blood vessels substantially equal in diameter. In addition, the anastomosis member can flexibly cope with a slight difference in size between the first and the second blood vessels.

The anastomosis member can be arranged on the outside of the first and the second blood vessels. Furthermore, the additional blood vessel can be arranged on the outside of the anastomosis member and fastened by a filament or a strap in at least one position. With this structure, it is possible to exert a greater engaging force and to assure a sufficient fixing force. By the use of the additional blood vessel, it is possible to effectively prevent the blood leakage out of the anastomosed site.

The anastomosis member can be compressed and expanded by changing the angles of the angled portions of the connecting members. The plate member has a stress-strain characteristic different from that of the connecting member and is flexible and low in rigidity.

Finally, the anastomosis member is readily deformable in correspondence to the curvatures of the first and the second blood vessels. Therefore, it is possible to uniformly apply the pressure to the first and the second blood vessels without causing local concentration of the pressure.

## Claims

1. An anastomosis member (11 to 18, 111) to be arranged at an anastomosed site of first and second blood vessels (33, 34) to carry out the anastomosis of said first and said second blood vessels (33, 34) ;
said anastomosis member (11-18, 111) having a generally cylindrical body comprising at least one plate member (11a to 14a) to be brought into contact with both of said first and said second blood vessels (33, 34) ;
said plate member having a plurality of protrusions (11b to 14b, 111b) formed on at least one of opposite surfaces thereof to be engaged with at least one of said first and said second blood vessels (33, 34) so as to prevent the dislocation of said first and said second blood vessels at said anastomosed site;
**characterized in**
**that** said anastomosis member (11 to 18, 111) has a stress-strain characteristic including a plurality of different kinds of regions, one kind of region corresponding to a low-rigidity part (140) deformable along the curvatures of said first and said second blood vessels (33, 34) to be tightly fitted thereto and another kind of region corresponding to a spring region (14c) which is compressible and self-expandable in diameter; and
**that** said generally cylindrical body comprises a plurality of said plate members forming said low-rigiding part.

2. An anastomosis member as claimed in claim 1, said anastomosis member (11 to 18) having a plurality of said generally cylindrical bodies and at least one connecting member (14c to 18c) connecting said generally cylindrical bodies to one another.

3. An anastomosis member as claimed in claim 1 or 2, wherein said anastomosis (11 to 18, 111) member has an elasticity so as to be compressed and expanded in diameter.

4. An anastomosis member as claimed in one of claims 1 to 3, wherein said plate member is made of a stainless steel plate or a shape memory alloy selected from a TiNi alloy and a beta Ti alloy.

5. An anastomosis member as claimed in one of claims 1 to 4, wherein said generally cylindrical body comprises at least one plate member (12a) wound into a helical shape.

6. An anastomosis member as claimed in one of claims 1 to 4, wherein said plate members forming said low-rigidity part are connected to one another in a zigzag pattern (11a) or in a lattice pattern (13a) or in a rhombic pattern (13a) or arranged in parallel to one another and a plurality of connecting members (14c) connecting said plate members (14a) to one another, said connecting members forming said spring region preferably being an elastic wire member.

## Patentansprüche

1. Anastomoseelement (11-18, 111), das an einer Anastomosestelle erster und zweiter Blutgefäße (33, 34) zum Durchführen der Anastomose der besagten ersten und zweiten Blutgefäße (33, 34) anzuordnen ist;
wobei das Anastomoseelement (11-18, 111) einen mindestens ein Plattenelement (11a-14a) umfassenden, im Allgemeinen zylindrischen Körper aufweist, um sowohl mit dem ersten als auch dem zweiten Blutgefäß (33, 34) in Kontakt gebracht zu werden; wobei das Plattenelement eine Vielzahl von Vorsprüngen (11b-14b, 111b) aufweist, die auf mindestens einer von entgegengesetzten Oberflächen davon zum Eingriff mit mindestens einem der ersten und zweiten Blutgefäße (33, 34) gebildet sind, um so die Versetzung der ersten und zweiten Blutgefäße an der besagten Anastomosestelle zu verhindern;
**dadurch gekennzeichnet, dass** das Anastomoseelement (11-18, 111) eine Belastungs/Dehnungs-Charakteristik aufweist, einschließlich einer Mehrzahl unterschiedlicher Bereichsarten, wobei eine Bereichsart einem Teil niedriger Steifigkeit (14a)- deformierbar entlang von Wendungen der ersten und zweiten Blutgefäße (33, 34), um dicht daran angepasst zu sein - entspricht und eine andere Bereichsart einem federnden Bereich (14c) entspricht, der in Bezug auf den Durchmesser komprimierbar und selbstaufweitbar ist; und
dass der im Allgemeinen zylindrische Körper eine Mehrzahl von Plattenelementen umfasst, die den Teil niedriger Steifigkeit bilden.

2. Anastomoseelement wie im Anspruch 1 beansprucht, wobei das Anastomoseelement (11 bis 18) eine Mehrzahl der im Allgemeinen zylindrischen Körper und mindestens ein Verbindungselement (14c-18c), der die im Allgemeinen zylindrischen Körper miteinander verbindet, aufweist.

3. Anastomoseelement wie im Anspruch 1 oder 2 beansprucht, wobei das Anastomoseelement (11 bis 18, 111) eine Elastizität aufweist, um in Bezug auf den Durchmesser komprimiert beziehungsweise aufgeweitet zu sein.

4. Anastomoseelement wie in einem der Ansprüche 1 bis 3 beansprucht, wobei das Plattenelement aus einer Edelstahlplatte oder einer Formerinnerungslegierung, ausgewählt aus einer TiNi-Legierung und einer beta-Ti-Legierung, gefertigt ist.

5. Anastomoseelement wie in einem der Ansprüche 1 bis 4 beansprucht, wobei der im Allgemeinen zylindrische Körper mindestens ein, zu einer helikalen Form gewundenes Plattenelement (12a) umfasst.

6. Anastomoseelement wie in einem der Ansprüche 1 bis 4 beansprucht, wobei die den Teil niedriger Steifigkeit bildenden Plattenelemente in einem Zick-Zack-Muster (11a) oder einem Gittermuster (13a) oder in einem rombischen Muster (13a) miteinander verbunden sind oder parallel zueinander angeordnet sind, mit einer Mehrzahl von die Plattenelemente (14a) miteinander verbindenden Verbindungselementen (14c), wobei die den federnden Teil bildenden Verbindungselemente vorzugsweise ein elastisches Drahtelement sind.

## Revendications

1. Elément d'anastomose (11 à 18, 111) destiné à être installé au niveau d'un site anastomosé de premier et second vaisseaux sanguins (33, 34) pour réaliser l'anastomose des premier et second vaisseaux sanguins (33, 34) ;
l'élément d'anastomose (11 à 18, 111) ayant un corps généralement cylindrique comprenant au moins un élément de plaque (11a à 14a) devant être amené en contact à la fois avec les premier et second vaisseaux sanguins (33, 34) ;
l'élément de plaque ayant une pluralité de saillies (11b à 14b, 111b) formées sur l'une au moins de ses surfaces opposées pour se raccorder avec l'un au moins des premier et second vaisseaux sanguins (33, 34) afin d'empêcher le déboîtement des premier et second vaisseaux sanguins au niveau du site d'anastomose,
**caractérisé en ce que**
l'élément d'anastomose (11 à 18, 111) a une caractéristique de contrainte-déformation comprenant une pluralité de types de régions différents, un type de région correspondant à une partie (14a) faiblement rigide déformable le long des courbes des premier et second vaisseaux sanguins (33, 34) pour s'emboîter étroitement avec ceux-ci, et un autre type de région correspondant à une région élastique (14c) dont le diamètre peut se comprimer ou s'élargir automatiquement, et
le corps généralement cylindrique comprend une pluralité des éléments de plaque formant la partie faiblement rigide.

2. Elément d'anastomose selon la revendication 1,
l'élément d'anastomose (11 à 18) ayant une pluralité de corps généralement cylindriques et au moins un élément de raccordement (14c à 18c) raccordant les corps généralement cylindriques les uns aux autres.

3. Elément d'anastomose selon la revendication 1 ou 2,
dans lequel l'élément d'anastomose (11 à 18, 111) possède une élasticité pour pouvoir être comprimé et élargi en diamètre.

4. Elément d'anastomose selon l'une des revendications 1 à 3,
dans lequel l'élément de plaque est fabriqué à partir d'une plaque en acier inoxydable ou d'un alliage à mémoire de forme sélectionné parmi un alliage de TiNi et un alliage de Ti bêta.

5. Elément d'anastomose selon l'une des revendications 1 à 4,
dans lequel le corps généralement cylindrique comprend au moins un élément de plaque (12a) enroulé selon une forme hélicoïdale.

6. Elément d'anastomose selon l'une des revendications 1 à 4,
dans lequel les éléments de plaque formant la partie faiblement rigide sont raccordés les uns aux autres selon une configuration en zigzag (11a) ou selon une configuration en treillis (13a) ou selon une configuration rhombique (13a), ou sont agencés parallèlement les uns aux autres, et une pluralité d'éléments de raccordement (14c) raccordant les éléments de plaque (14a) les uns aux autres, forment la région élastique, en étant de préférence un élément métallique élastique.
